# EUROPÄISCHE PATENTSCHRIFT

(11) **EP 1 345 027 B1**
(45) Veröffentlichungstag und Bekanntmachung des Hinweises auf die Patenterteilung: **14.12.2005**
(21) Anmeldenummer: 03000146.5
(22) Anmeldetag: 03.01.2003
(51) Int. Cl.: G01N 27/12, G01N 27/04, G01N 33/46

(54) **Vorrichtung zur Bestimmung der Holzfeuchte in Holzstapeln**
Apparatus for measuring wood humidity in lumber piles
Dispositif de détermination de l'humidité du bois dans piles de planches

(30) Priorität: 15.03.2002 CL 51902
(43) Veröffentlichungstag der Anmeldung: 17.09.2003
(73) Patentinhaber: Neumann, Rodolfo J., Prof. Dr.-Ing., Concepcion (CL)
(72) Erfinder: Neumann, Rodolfo J., Prof. Dr.-Ing., Concepcion (CL)
(74) Vertreter: Behrens, Dieter

(56) Entgegenhaltungen:
- US-A1- 2002 067 173
- DATABASE WPI Section EI, Week 199618 Derwent Publications Ltd., London, GB; Class S03, AN 1996-178616 XP000150126 -& RU 2 042 130 C (KOLGANOV V A), 20. August 1995 (1995-08-20)
- DATABASE WPI Section EI, Week 198918 Derwent Publications Ltd., London, GB; Class S03, AN 1989-137146 XP000104557 -& SU 1 436 046 A (BELORUSSIAN POLY), 7. November 1988 (1988-11-07)
- DATABASE WPI Section EI, Week 200236 Derwent Publications Ltd., London, GB; Class S03, AN 2002-327407 XP000256713 -& RU 2 180 746 C (URALDREV-INTO CO LTD), 20. März 2002 (2002-03-20)

## Beschreibung

Die Erfindung betrifft eine Vorrichtung für die Bestimmung der Holzfeuchte in Holzstapeln, in denen die Holzbalken oder -bretter in durch Trennleisten voneinander getrennten Holzlagen gestapelt sind. Diese Vorrichtung arbeitet nach dem Verfahren der elektrischen Widerstands-oder Impedanzmessung und ist mit mindestens einem Paar Elektroden versehen, über die der elektrische Messstrom in das bzw. aus dem Holz geleitet wird und wird insbesondere bei der Trocknung von Holzbrettern in Trocknungsanlagen eingesetzt.

Zur Trocknung von in Balken, Bretter o.dgl. aufgeschnittenem Holz wird hauptsächlich die Konvektionstrocknung eingesetzt, bei der warme Luft über das feuchte Holz strömt, dem Holzstapel Wärme zuführt und von diesem die verdampfte Feuchtigkeit aufnimmt. Dadurch trocknet die Holzoberfläche eher als der Kern. Weil das Holz ab Erreichen einer Feuchte von nur noch etwa 30% zu schwinden beginnt, können die während der Trocknung aufgebauten Feuchte-Gradienten im Holz zu Rissen führen. In konventionellen Trocknungsverfahren werden diese Risse dadurch vermieden, dass man den Trocknungsvorgang bei hohen relativen Luftfeuchtigkeiten beginnt. Um die Trockenzeiten nicht unnötig zu verlängern, kann mit abnehmender Holzfeuchtigkeit die Lufttemperatur erhöht und dessen relative Feuchtigkeit vermindert werden. So entsteht die Notwendigkeit, die Holzfeuchtigkeit kontinuierlich zu messen und die Trocknungsbedingungen entsprechende dem Trocknungsfortschritt zu regeln, insbesondere im Fasersättigungsbereich und darunter.

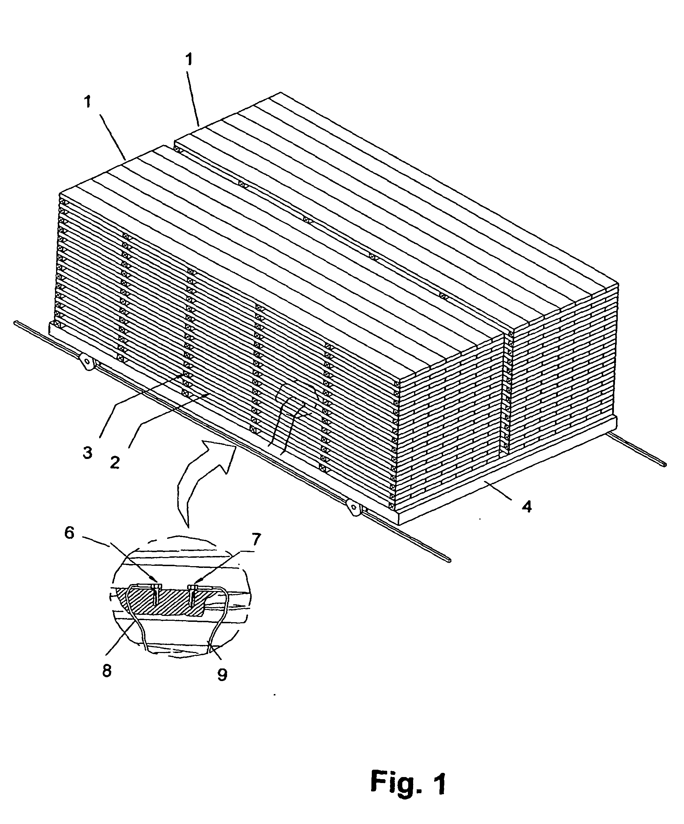

Damit die Luft das Holz gut umströmen kann, werden die lagenweise zu Holzstapeln 1 gestapelten Balken oder Bretter 2 mit rechteckförmigen Stapelleisten 3 voreinander getrennt, wie das Fig. 1 zeigt. In leistungsfähigen Trocknern wird das Holz auf Wagen 4 gestapelt, die in einen entsprechend großen Trockner gefahren werden. Auf dem Wagen 4 sind nebeneinander 1 bis 4, meist 2 Holzstapel und hintereinander bis zu 4 oder 5 Holzstapeln gestapelt. Es werden auch jeweils bis zu 4 Stapel übereinander vorgesehen. Die warme Luft strömt seitlich in die Stapel ein. Sie kühlt sich durch die Wärmeabgabe in Strömungsrichtung ab. Um zu vermeiden, dass die in der Nähe der ausströmenden Luft gelegenen Bretter langsamer trocknen, wird die Luftströmungsrichtung in periodischen Zeitabständen umgekehrt. Weil die Lufttemperatur und damit die Trocknungsrate in Strömungsrichtung exponentiell abnimmt, trocknet nun das Holz nahe der Innenseiten der beiden in Fig. 1 dargestellten Holzstapel am langsamsten. Es besteht also die Notwendigkeit, die Holzfeuchtigkeit in den Brettern, insbesondere in den von den Seitengängen zwischen Trockneraußenwand und Holzstapel am entferntesten gelegenen Brettern, zu messen. Die Seitengänge haben eine Breite von etwa 0,8 bis 2 m, sind aber meist nur 1,0 bis 1,2 m breit.

Nach dem Stand der Technik wird die Holzfeuchte u.a. über die Messung des elektrischen Widerstandes zwischen zwei Elektroden bestimmt, die hierzu in das Holz eingebracht werden. Fig. 1 zeigt die üblichen Elektroden 6 und 7, die entweder in das Holz genagelt oder in vorgebohrte Löcher getrennt oder gemeinsam getrieben werden, und die über Kabel 8 und 9 und Verbindern mit einem geeigneten Messinstrument verbunden werden (DE 43 19 603 A1). Die Messmethode bestimmt den geringsten elektrischen Widerstand zwischen den beiden Elektroden 6 und 7, der an der feuchtesten Stelle gegeben ist. Die Korrelationen zwischen dem Widerstand und der Holzfeuchte sind für die meisten kommerziellen Holzarten bekannt und werden zum Teil je nach der Temperatur korrigiert. Da während der Trocknung die Feuchtigkeit nach außen abnimmt, wird somit meistens die Feuchtigkeit an der Spitze der Elektroden bestimmt, wo die Eindringtiefe am größten ist. Dies hat einen praktischen Wert, da, um Trocknungsschäden zu vermeiden, meistens das Klima in der Kammer erst verschärft werden kann, wenn die höchste Holzfeuchtigkeit bestimmte Werte erreicht hat.

Obgleich die Bestimmung der Holzfeuchte über den elektrischen Widerstand die bekannteste Messmethode ist, hat sie den Nachteil, dass sie sehr aufwendig ist: die Elektroden 6 und 7 müssen zuerst während der Stapelung in das Holz getrieben werden. Nachdem das Holz in die Trockenkammer gefahren worden ist müssen die Elektroden an dort vorhandene Kabel angeschlossen werden. Nach der Trocknung müssen die Kabel wieder entfernt werden und schließlich müssen die Elektroden während der Entstapelung von den Holzbrettern wieder abgetrennt werden. Wenn automatische Stapel- und Entstapelungsanlagen benutzt werden, ist der Aufwand desto größer. Da eine Holzcharge mehrere tausend Bretter haben kann, ist es üblich, bis zu 10 Messpunkte pro Holzcharge vorzusehen, was den Aufwand nochmals erhöht. Es ist zu beachten, dass man normalerweise in mit Wagen beladenen Trocknern nur einen guten Zugang zu Brettern hat, die in der Nähe der Seitengänge gestapelt sind. Zu den innen gelegenen Brettern hat man normalerweise keinen Zugang und dort können auch Kabel 8 und 9 nicht ohne weiteres angeschlossen werden, weswegen dort die Holzfeuchte nur mit noch größerem Aufwand, wenn überhaupt, gemessen werden kann. Weiterhin ist nachteilig, dass die vorgebohrten Löcher das Holz beschädigen und dass öfters nicht alle Nägel oder Elektroden nach der Trocknung entfernt werden und diese dadurch größere Schäden in den nachfolgenden Hobelmaschinen anrichten können.

Besonders bei schnell trocknenden Kieferhölzern ist der Aufwand mit den einzubringenden Elektroden zu groß und kommt zu teuer. Um dennoch die Widerstandsmethode einsetzen zu können, werden nach einem anderen bekannten Verfahren einzelne Stapelleisten zwischen den Holzlagen durch metallische im Querschnitt ebenfalls rechteckförmige Elektrodenstäbe ersetzt, was auch mit höherem Aufwand verbunden ist.

Bei der Messvorrichtung nach der russischen Patentschrift RU 2 042 130 C1 ist für die Messung ein Paar Elektroden vorgesehen, die jeweils aus einem Stab mit meist diametral abstehenden blattförmigen, gegebenenfalls kammartigen blattförmigen Kontaktteilen versehen sind, die aus leitendem Material, z.B. Messing, bestehen. Der Elektrodenstab kann mehrere blattförmige Kontakteile haben, um einen guten Kontakt mit dem Sägemehl auf den Holzlagen zu bewirken. Die paarweise nebeneinander zwischen die Holzlagen eingeführten Elektroden werden zum Messung um 90° gedreht und nehmen dann an den Enden der Kontaktteile eine abgebogene Form an, liegen also flächig auf der Oberfläche der Holzlagen an. Die Feuchtigkeltsmessung ist eine Widerstandsmessung der trockeneren Oberflächenfeuchte zwischen den Elektroden. Mit den genannten Messmethoden werden im wesentlichen die Oberflächenfeuchte des Holzes und nicht die Kernfeuchte bestimmt.

Mit wiederum anderen Messmethoden wird die elektrische Kapazität des Holzes gemessen (WO 96/28 741) oder das Verhalten gegenüber Mikrowellen. Beide Methoden haben andere Nachteile wie z.B. die Abhängigkeit von der Rohdichte des Holzes, die nur schwer zu erfassen ist.

Die sowjetische Patentanmeldeschrift SU 1436046 A1 offenbart eine Messvorrichtung zur Bestimmung der Holzfeuchte in Holzstapeln mit nadelförmigen Elektroden, die durch einen Hebemechanismus senkrecht in das Holz eingetrieben werden. Der Hebemechanismus besteht hauptsächlich aus zwei Antriebsrollen, die durch entsprechende Handgriffe betätigt werden. Um den Messaufwand und die damit verbundenen Mehrkosten zu reduzieren, sollte ein neues Widerstands-Messverfahren mit Elektroden, wenn möglich, folgende Bedingungen erfüllen:

Die Elektroden sollten leicht an den für die Messung günstigsten Stellen in das Holz getrieben werden können. Die Elektroden sollten während der Trocknung von einem trockeneren Ort zu einem feuchteren Ort gewechselt werden können. Es sollte ein guter Kontakt zwischen der Elektrode und dem Holz gewährleistet werden. Die Elektroden sollten durch das Eindringen in das Holz hier wenig Schaden anrichten. Es sollte die Möglichkeit ausgeschlossen werden, dass die Elektroden nach der Trocknung nicht vollständig entfernt werden. Die Elektroden sollten die Kernfeuchte der gestapelten Bretter erfassen können. Die Messmethode sollte in möglichst vielen Brettern die feuchtesten Stellen erfassen, auch an solchen nur schlecht zugänglichen Bereichen. Die Messvorrichtung sollte kostengünstig und sehr zuverlässig sein.

Aufgabe der Erfindung ist es, eine Widerstands-Messvorrichtung aufzuzeigen, welche die vorerwähnten Bedingungen auf einfache Weise erfüllt.

Diese Aufgabe wird durch die mit ihren Ausgestaltungen in den Patentansprüchen gekennzeichnete Widerstands-Messvorrichtung in überraschend einfacher Weise gelöst.

Durch die Gestaltung mit abstehenden Kontaktteilen, Vorsprüngen, Schneiden, Messern oder Zähnen vorgesehene Ausbildung der stabförmigen Elektroden ist es möglich, sie lang auszubilden, so dass sie über die ganze Stapelbreite reichen können und ihre leichte Einführbarkeit in den Luftspalt zwischen den gestapelten Holzbrettern gesichert ist. Danach muss die Elektrode lediglich um ihre Längsachse gedreht werden, um in guten Eingriff mit dem feuchten Holz zu kommen. Nach Anschluss der beiden Elektroden an das Messinstrument, das die Stromquelle enthält, kann der elektrische Widerstand zwischen zwei solchen Elektroden in bekannter Weise bestimmt werden kann. Umgekehrt kann durch eine Rückdrehung der Elektrode diese leicht wieder aus dem Holzstapel entfernt werden. Die asymmetrisch oder diametral angeordneten Schneiden oder Spitzen an dem Stab werden durch Drehung des Stabes in das Holz getrieben werden. Die Messung der Kernfeuchte ist so bequem möglich.

Ausführungsbeispiele der Erfindung werden anhand einer Zeichnung näher erläutert, in der zeigt:
Fig. 1 zwei nebeneinander angeordnete Holzstapel mit bekannten, genagelten Elektroden;
Fig. 2 Holzstapel mit zwei erfindungsgemäßen Elektroden, die an drei axial versetzten Stellen je zwei diametral angeordnete Schneiden zum Eindringen in das Holz tragen;
Fig. 3 eine erfindungsgemäße Elektrode nach Fig. 2 in vergrößertem Maßstab, bei welcher diese als elliptisches, polygonales Rohr mit am einen Ende vorgesehenen abstehenden Hebeln zum Verdrehen der Elektrode ausgebildet ist;
Fig. 4 eine erfindungsgemäße Elektrode mit zwei diametral angeordneten Spitzen am vorderen Ende;
Fig. 5 eine Doppel-Elektrode mit zwei konzentrisch zueinander angeordnete Elektroden mit jeweils zwei diametral abstehenden Spitzen,
Fig. 6 eine Doppel-Elektrode mit zwei konzentrisch zueinander angeordnete Elektroden, die jeweils nur eine Spitze haben, wobei diese diametral entgegengesetzt ausgerichtet sind, und
Fig. 7 eine mit einem wärmeisolierenden Mantel umgebene Elektrode mit Betätigungshebeln und zwei diametral angeordneten Spitzen.

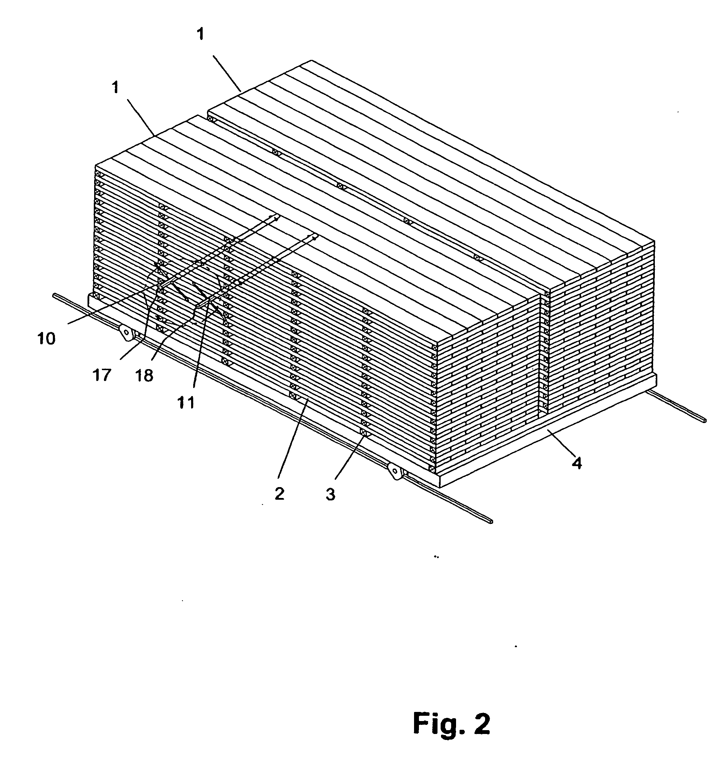

In Fig.2 sind zwei Elektroden 10 und 11 als elliptisches Rohr 13 (verschlossen dargestellt) mit einem oder zwei radial abstehenden Hebeln 14 gezeigt, die in einer gewissen Entfernung voneinander in einem Spalt zwischen den gestapelten Brettern angebracht werden. Um die Kernfeuchte zu erfassen, ist es erforderlich, dass die Elektroden mindestens je eine Schneide 21 und 22 haben. Da der Durchmesser h1 kleiner ist als die Dicke der Stapelleisten, kann das elliptische Rohr 13 leicht in den Spalt zwischen zwei Holzbretterlagen eingeschoben werden. Durch eine Verdrehung der Elektroden über die Hebel 14 um meist etwa 90° dringen die Schneiden in das Holz ein, weil der Durchmesser h2 etwas größer ist als die Stapelleistendicke, wie dies Fig. 2 zeigt. Der elektrischen Widerstand des Holzes zwischen den Elektroden wird bestimmt, in dem elektrischer Strom von einem geeigneten Messinstrument über elektrische Kabel 17 und 18 in die Elektroden und von diesen in das Holz eingeleitet bzw. abgeleitet wird. Die Kabel sind z.B. mit den Elektroden 10, 11 fest verschraubt, oder tragen Steckverbinder, z.B. Bananenstecker 16, die in Anschlussbuchsen oder -bohrungen in den Elektroden eingesteckt werden. Der Widerstand zwischen beiden Elektroden wird vom feuchtesten Weg des Holzes bestimmt, das zwischen den Schneiden der Elektroden gelegen ist.

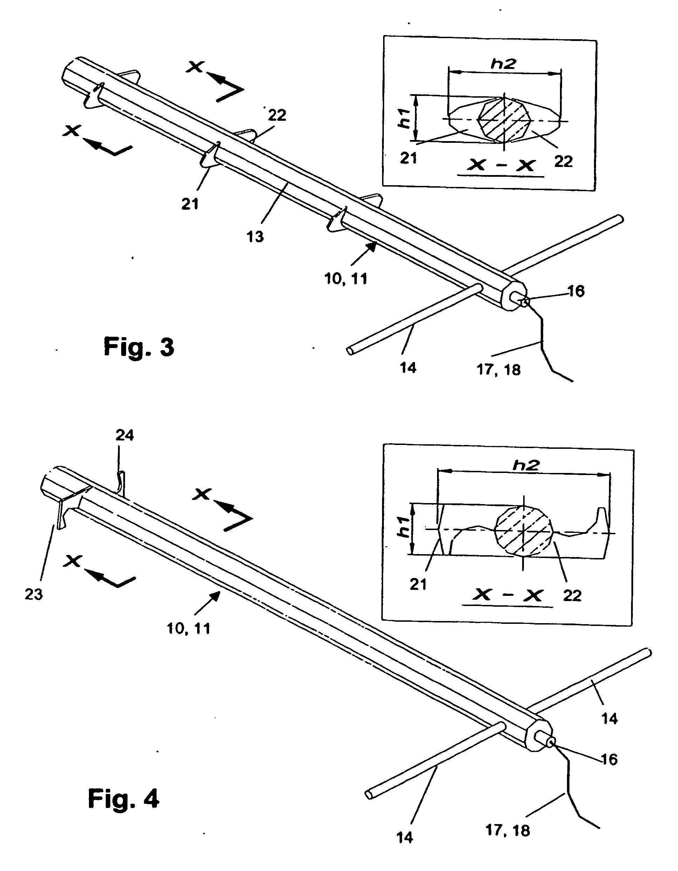

Es können auch mehrere Schneiden an einer Elektrode vorgesehen sein. Figur 3 zeigt Elektroden 10, 11 mit 6 halbelliptischen Schneiden 21 und 22. Mit zwei dieser Elektroden kann die höchste Kernfeuchtigkeit in mehreren Brettern bestimmt werden kann. An Stelle der Hebel 14 kann jede Elektrode mit einem Kupplungsanschluss, z.B. in Form eines zylindrischen oder sechseckigen oder sechskantförmigen Zapfens versehen werden, der es ermöglicht, die Elektrode mit einer elektrischen Bohrmaschine, z. B. mit eingespanntem Sechskant-Inbusschlüssel, oder einem Elektroschrauber zu drehen. In diesem Fall kann der elektrische Anschluss der Kabel weiterhin mit einem Steckverbinder wie einem Bananenstecker erfolgen, der in die Bohrung gesteckt wird.

Um die Kernfeuchte in nur zwei Brettern gleichzeitig zu bestimmen, kann jede Elektrode 10, 11 an der Spitze mit nur zwei in entgegengesetzte Richtungen abstehenden Spitzen 23 und 24 versehen sein, wie diese in Fig. 4 gezeigt sind. Hier wird auch die Mindesthöhe h1 gezeigt, die kleiner sein muss als die Dicke der Stapelleisten. Auch hier dringen die Spitzen 23, 24 oder Schneiden durch Verdrehung der Elektrode mit dem Handhebel 14 in das Holz ein, wobei die Eindringtiefe von der Höhe h2 bestimmt wird. Wenn die zweite Elektrode genauso angebracht ist, wird die größte Feuchtigkeit bis zu dieser Tiefe im Holz gemessen.

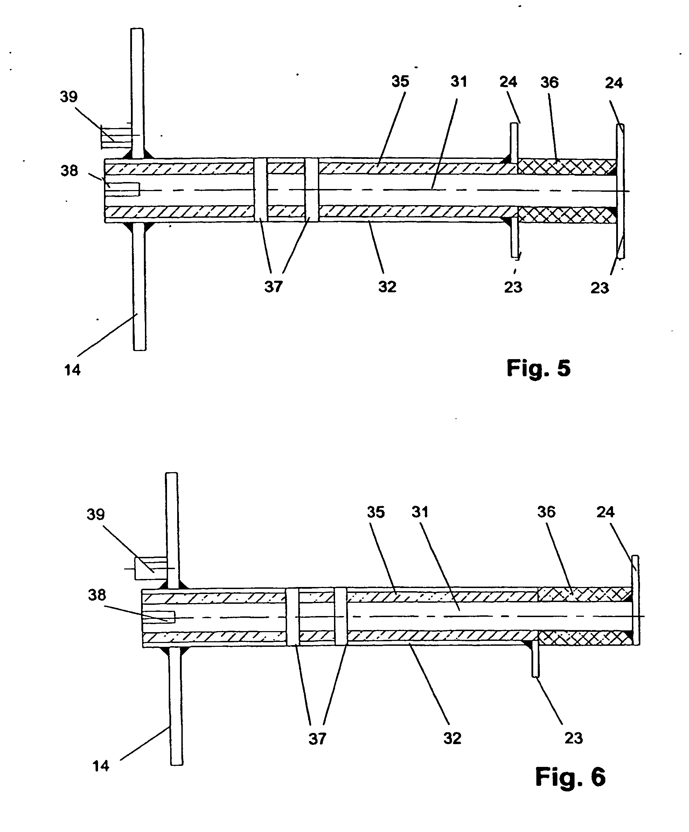

Ein Paar Elektroden kann auch derart ausgebildet sein, wie dies Fig. 5 zeigt, wonach eine stabförmige Elektrode 31 innerhalb einer rohrförmigen Elektrode 32 aufgenommen ist. Bei einer solchen Doppel-Elektrode ist die stabförmige Elektrode 31 nur am vorderen Ende mit Spitzen 23, 24 ausgebildet. Sie ist von einer rohrförmigen Elektrode 32 aufgenommen und umschlossen, welche kürzer ist als die stabförmige Elektrode 31 und von ihr durch einen Isolationsmantel 35 elektrisch getrennt ist. Auch sie trägt nur an ihrem vorderen Ende Spitzen 23, 24 oder auch Schneiden. Die stabförmige Elektrode 31 ist auch am freien vorderen Ende mit einem elektrisch und thermisch isolierenden Mantel 36 umgeben, welcher jedoch hauptsächlich hinsichtlich guter Wärmeisolation ausgebildet ist. Um beide Elektroden mit dem Hebel 14 gleichzeitig verdrehen zu können, kann der Stab der Elektrode 31 und das Rohr der Elektrode 32 mit einem oder mehreren Querbolzen 37 miteinander verbunden sein, die aus elektrisch isolierendem Material bestehen. Die elektrische Verbindung der Elektroden mit dem Messinstrument kann z.B. über Bananenstecker erfolgen, die in eine zentrale Anschlussbuchse bzw. -bohrung 38 in der stabförmigen Elektrode 31 und in einer seitliche Anschlussbuchse 39 am Betätigungshebel 14 der rohrförmigen Elektrode 32 gesteckt werden.

Es kann von Vorteil sein, den elektrischen Widerstand in zwei Teilstücken von übereinander gestapelten Brettern in Serie geschaltet zu messen. Hierzu werden zwei Elektroden verwendet und zwar eine Einzel-Elektrode nach Fig. 3, 4 oder 6 ohne elektrischen Anschluss mit jeweils zwei diametral entgegengesetzt abstehenden Spitzen oder Schneiden und - in einer bestimmten horizontalen Entfernung - eine Doppel-Elektrode nach Fig. 6, die gegenüber der Doppel-Elektrode nach Fig. 5 dahingehend abgewandelt ist, dass die innere Elektrode 31 und die äußere Elektrode 32 jeweils nur eine radial abstehende Spitze 23. bzw. 24 (oder Schneide) haben und diese beiden Spitzen diametral entgegengesetzt abstehen. Die beiden Elektroden werden nebeneinander in den Zwischenraum zwischen zwei übereinander gestapelten Brettern eingebracht und zwar so, dass die Spitzen 23 und 24 (oder Schneiden) der beiden Elektroden einen axialen Abstand voneinander haben. Der Mess-Strom wird von der Anschlussbuchse 38 über die nur eine Spitze 24 der inneren stabförmigen Elektrode 31 in das eine Brett eingeleitet und über die nur eine Spitze 23 der äußeren rohrförmigen Elektrode 32 der Doppel-Elektrode nach Fig. 6 über die Anschlussbuchse 39 aus dem darrüberliegenden anderen Brett abgeleitet. Der Mess-Strom fließt längs des einen Bretts zu einer Spitze oder Schneide der Einzel-Elektrode und tritt über deren diametrale andere Spitze oder Schneide in das andere Brett über und fließt in diesem zu der nur einen Spitze 23 der rohrförmigen Elektrode 32 der Doppel-Elektrode zurück. Somit wird im unteren und oberen Brett der elektrische Widerstand der jeweiligen Teilstücke zwischen den Elektrodenspitzen erfasst.

Bei entsprechendem Abstand der Anschlussbuchsen 38 und 39 der Doppel-Elektrode können die Messleitungen in einem Doppelstecker zusammengefasst werden. Durch die Verwendung der Doppel-Elektrode nach Fig. 6 in Verbindung mit einer Einzel-Elektrode nach Fig. 3, 4 oder 6 kann der Messweg über zwei Bretter getrennt verlaufen und kann die Länge des Mess- / 6 wegs frei bestimmt werden.

Diese Anordnung ist auch besonders vorteilhaft, um den Messwert von einem akkubetriebenen Sender ohne Kabel an einen entfernten Empfänger zu senden, da beide Anschlussbuchsen nahe beieinander liegen und somit auch ein einzige Sender für beide Anschlüsse eingesetzt werden kann.

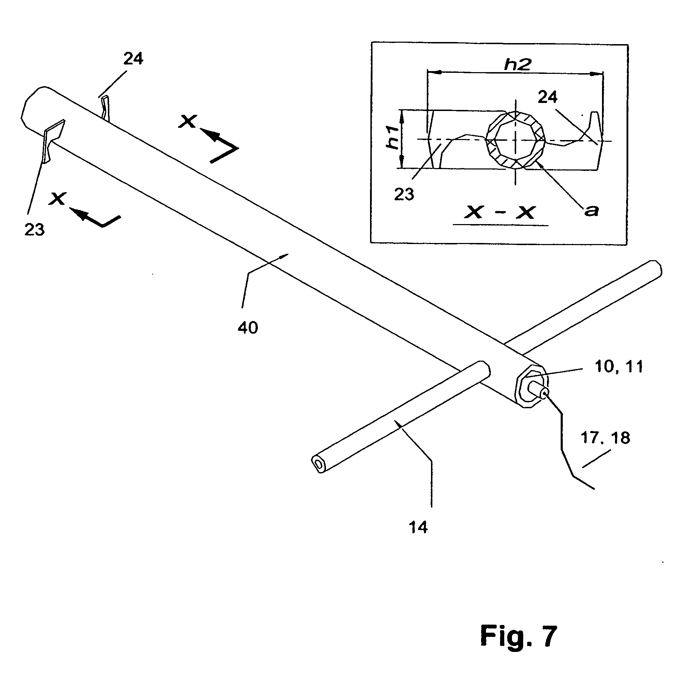

Die Wärmeleitung von der Elektrode über die Spitzen oder Schneiden in das Holz kann zu einer vorzeitigen Austrocknung des Holzes führen. Dies ist vermieden, wenn die Elektroden 10, 11 mit einem wärmeisolierenden Mantel 40 umgeben sind, wie er in Fig. 7 gezeigt ist. Die Wärmeleitung in das Holz wird weiter verringert, wenn auch der Hebel 14 wärmeisoliert wird.

## Patentansprüche

1. Vorrichtung für die Bestimmung der Holzfeuchte in Holzstapeln bei der Trocknung in Trocknungsanlagen, in denen die Holzbalken oder -bretter in durch Trennleisten voneinander getrennten Holzlagen gestapelt sind, die nach dem Verfahren der elektrischen Widerstandsmessung bzw. Impedanzmessung arbeitet und mit mindestens einem Paar Elektroden versehen ist, über die der elektrische Mess-Strom in das Holz eingeleitet bzw. aus dem Holz abgeleitet wird, wobei jede Elektrode (10, 11; 31, 32) als massiver oder hohler, insb. rohrförmiger Stab mit einem oder mehreren radial abstehenden Kontakteilen ausgebildet ist, und in einer ersten Richtung senkrecht zur Längsachse eine Gesamtdicke (h1) aufweist, welche geringer als der Abstand zwischen den gestapelten Holzlagen ist, und in einer zweiten Richtung, ebenfalls senkrecht zur Längsachse mit den Kontaktteilen eine Gesamtdicke (h2) hat, die größer als der Abstand zwischen den gestapelten Holzlagen ist,
**dadurch gekennzeichnet, dass**
jeder Kontaktteil als etwa rechtwinklig zur Achse abstehende Schneide (21, 22) oder Spitze (23, 24) ausgebildet ist und dass jede Schneide oder Spitze der zwischen die Holzlagen eingeführten Elektroden durch deren Verdrehen in das Holz hineingetrieben werden kann.

2. Vorrichtung nach Anspruch 1, **dadurch gekennzeichnet, dass** jede Elektrode (10, 11) ein oder mehrere axial voneinander getrennte Paare von diametral zueinander gleich radial abstehenden Schneiden (21, 22) hat, welche durch Verdrehen der Elektrode gleichzeitig in ein oder mehrere Bretter eingetrieben werden können, die über und unter dem Spalt zwischen zwei Holzlagen gestapelt sind.

3. Vorrichtung nach Anspruch 1, **dadurch gekennzeichnet, dass** eine stab- oder rohrförmige Elektrode (31) konzentrisch in einer rohrförmigen kürzeren Elektrode (32) unter Zwischenlage eines elektrisch isolierenden Mantels (35) angeordnet ist und dass jede Elektrode (31,32) am vorderen Ende je eine radial abstehenden Schneide oder Spitze (23, 24) hat, die beide axial voneinander getrennt sind und in der gleichen radialen Richtung angeordnet sind.

4. Vorrichtung nach einem der Ansprüche 1 bis 3, **dadurch gekennzeichnet, dass** jede Elektrode (10, 11) von einem wärmeisolierenden, die Wärmeleitung von der Luft über die Elektrode oder die vorstehenden Schneiden (21,22) oder Spitzen (23,24) in das Holz vermindernden Mantel (40) umgeben ist.

5. Vorrichtung nach einem der Ansprüche 1 bis 4, **dadurch gekennzeichnet, dass** am Ende der Elektroden ein mechanisches Kupplungsteil vorgesehen ist, über das ein elektromotorischer Drehantrieb für die Elektrode ankuppelbar ist.

## Claims

1. Apparatus for determining wood humidity in woodpiles during drying in a drying apparatus in which wooden beams or wooden boards are stacked in layers separated by slats, which measures in accordance with the method of electrical resistance or impedance measurement, and which comprises at least one pair of electrodes by means of which the electrical measuring-current is transmitted into the wood or out of it, respectively, each electrode (10, 11; 31, 32) made of a solid or hollow, especially tubular, rod with one or more radially protruding contact pieces and having in a first direction perpendicular to the longitudinal axis a total thickness (h1) which is smaller than the distance between stacked wooden layers, and having in a second direction, also perpendicular to the longitudinal axis, including the contact pieces a total thickness which is greater than the distance between wooden layers,
**characterized in that** each contact piece is formed as a bezel (21, 22) or spike (23, 24) directed perpendicular to the axis and that each bezel or spike of the electrodes is adapted to be pierced into the wood by rotation of the electrodes when inserted between wooden layers.

2. Apparatus according to claim 1, **characterized in that** each electrode (10, 11) comprises one or more spaced apart pairs of diametrically opposed similar radially protruding bezels (21, 22), which are adapted to be pierced at the same time into one or more boards which are stacked above and below the space between two wooden layers by rotation of the electrodes.

3. Apparatus according to claim 1, **characterized in that** a rod or tubular shaped electrode (31) is concentrically arranged within a shorter tubular shaped electrode (32) with a coating layer (35) of an electrically insulating material interposed between them, and that each electrode (31, 32) has a radially protruding bezel or spike (23, 24) at their front end which are spaced apart from each other and are oriented in the same radial direction.

4. Apparatus according to any of claims 1 to 3, **characterized in that** each electrode (10, 11) is covered by a heat insulating coating (40) which reduces heat conduction from the air through the electrode or the protruding bezels (21, 22) or spikes (23, 24) into the wood.

5. Apparatus according to any of claims 1 to 4, **characterized in that** there is provided at the one end of the electrodes a mechanical coupling piece adapted for coupling an electric motor for rotation of the electrodes.

## Revendications

1. Dispositif de détermination de l'humidité du bois dans des piles de planches lors du séchage dans des dispositifs de séchage dans lesquels les poutres en bois ou les planches sont empilées en couches séparées par deslattes, qui emploie la méthode de la mesure de la résistance respectivement de l'impédance électrique et qui comprend au moins une paire d'électrodes, par lesquelles le courant de mesure électrique est transmis dans ou du bois, avec chaque électrode (10, 11; 31, 32) en forme de tige massive ou creuse, notamment tubulaire, avec un ou plusieurs éléments de contact faisant radialement saillie, et ayant dans une première direction perpendiculaire à l'axe longitudinal une épaisseur globale (h1) inférieure à la distance entre les couches de planches empilées, et ayant dans une deuxième direction, également perpendiculaire à l'axe longitudinal avec les éléments de contact, une épaisseur globale (h2) supérieure à la distance entre les couches de planches empilées, **caractérisé en ce que**
chaque élément de contact monté de façon perpendiculaire par rapport à l'axe est en forme de lame (21, 22) ou de pointe (23, 24) et que chaque lame ou chaque pointe des électrodes introduites entre les couches de bois peut être enfoncée dans ledit bois par rotation des électrodes.

2. Dispositif selon la revendication 1, **caractérisé en ce que** chaque électrode (10, 11) comprend une ou plusieurs paires de lames, séparées axialement l'une de l'autre et montées diamétralement opposées et dans la même direction radiale (21, 22), qui peuvent simultanément être enfoncées dans une ou plusieurs planches empilées sur ou sous l'espace entre deux couches de bois.

3. Dispositif selon la revendication 1, **caractérisé en ce qu'**une électrode en forme de tige massive ou tubulaire est arrangée (31) de façon concentrique dans une électrode tubulaire plus courte (32) avec une gaines isolante électrique entre les deux, (35) et que chaque électrode (31,32) comprend à l'extrémité de devant une lame ou pointe radialement saillante (23, 24), qui sont séparées axialement et arrangées dans la même direction radiale.

4. Dispositif selon une des revendications 1 à 3, **caractérisé en ce que** chaque électrode (10, 11) est entourée d'une gaine isolante, réduisant la conduction thermique de l'air par l'électrode ou les lames (21,22) ou pointes (23,24) saillantes dans le bois (40).

5. Dispositif selon une des revendications 1 à 4, **caractérisé en ce qu'**au bout de chaque électrode est situé un moyen d'accouplement mécanique permettant l'accouplement d'un entraînement électromoteur pour la rotation de l'électrode.
